(19) Europäisches Patentamt
European Patent Office
Office européen des brevets

(11) Veröffentlichungsnummer: **0 465 901 A1**

# (12) EUROPÄISCHE PATENTANMELDUNG

(21) Anmeldenummer: **91110326.5**

(51) Int. Cl.[5]: **C07D 277/82**, A01N 43/78

(22) Anmeldetag: **22.06.91**

(30) Priorität: **07.07.90 DE 4021658**

(43) Veröffentlichungstag der Anmeldung:
**15.01.92 Patentblatt 92/03**

(84) Benannte Vertragsstaaten:
**BE CH DE DK ES FR GB IT LI NL SE**

(71) Anmelder: **BAYER AG**
**W-5090 Leverkusen 1 Bayerwerk(DE)**

(72) Erfinder: **Wagner, Klaus, Dr.**
**Jakob-Böhme-Strasse 2**
**W-5000 Köln 80(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) 2-Acylamino-7-chlorbenzthiazole.

(57) Neue 2-Acylamino-7-chlorbenzthiazole der Formel (I)

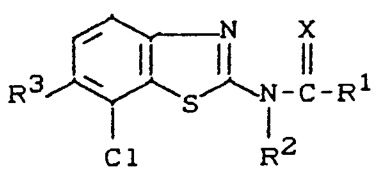

(I)

in welcher

- $R^1$ für Alkyl, Halogenalkyl, Alkoxyalkyl, Alkoxycarbonylalkyl, Cycloalkyl, Alkylthio, Alkylamino, Alkoxyalkylamino, Dialkylamino, für Cycloalkylamino oder für Amino steht, welches durch Cycloalkyl und durch Alkyl substituiert ist,
- $R^2$ für Wasserstoff, Alkyl oder Cycloalkyl steht,
- $R^3$ für Wasserstoff, Halogen oder Halogenalkyl steht und
- X für Sauerstoff oder Schwefel steht,

ein Verfahren zu ihrer Herstellung sowie ihre herbizide Verwendung.

EP 0 465 901 A1

Die Erfindung betrifft neue 2-Acylamino-7-chlorbenzthiazole, ein Verfahren zu ihrer Herstellung und ihre Verwendung als Herbizide.

Es ist bekannt, daß bestimmte 2-Acylaminobenzthiazole, wie beispielsweise die Verbindung N,N'-Dimethylbenzthiazol-2-ylharnstoff herbizide Eigenschaften besitzen (vgl. GB 1.085.430 oder K.H. Büchel "Pflanzenschutz und Schädlingsbekämpfung" S. 172; Georg Thieme Verlag Stuttgart 1977).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 2-Acylamino-7-chlorbenzthiazole der allgemeinen Formel (I),

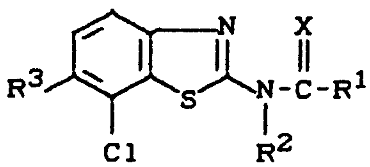

(I)

in welcher

- $R^1$ für Alkyl, Halogenalkyl, Alkoxyalkyl, Alkoxycarbonylalkyl, Cycloalkyl, Alkylthio, Dialkylamino oder für Amino steht, welches durch Cycloalkyl, Alkyl, Alkoxyalkyl, Halogenalkyl, Alkenyl, Alkinyl und/oder Alkoxycarbonylalkyl substituiert ist,
- $R^2$ für Wasserstoff, Alkyl oder Cycloalkyl steht,
- $R^3$ für Wasserstoff, Halogen oder Halogenalkyl steht und
- X für Sauerstoff oder Schwefel steht,

gefunden.

Weiterhin wurde gefunden, daß man die neuen 2-Acylamino-7-chlorbenzthiazole der allgemeinen Formel (I),
erhält, wenn man 2-Amino-7-chlorbenzthiazole der Formel (II),

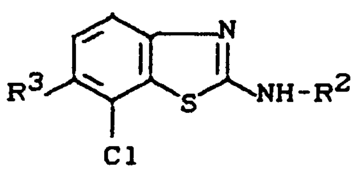

(II)

in welcher
$R^2$ und $R^3$ die oben angegebene Bedeutung haben,
mit Acylhalogeniden der Formel (III),

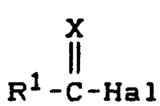

(III)

in welcher
Hal für Halogen steht und
$R^1$ und X die oben angegebene Bedeutung haben,
oder mit Iso(thio)cyanaten der Formel (IV),

$R^{1-1}$-N = C = X (IV)

in welcher
$R^{1-1}$ für Alkyl steht und
X die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen 2-Acylamino-7-chlorbenzthiazole der allgemeinen Formel (I) herbizide Eigenschaften besitzen.

Überraschenderweise zeigen die erfindungsgemäßen 2-Acylamino-7-chlorbenzthiazole der allgemeinen Formel (I) eine deutlich bessere herbizide Wirksamkeit bei vergleichbar guter Nutzpflanzenselektivität als die aus dem Stand der Technik bekannten 2-Acylaminobenzthiazole, wie beispielsweise die Verbindung N,N'-Dimethylbenzthiazol-2-ylharnstoff, welche chemisch und wirkungsmäßig naheliegende Verbindungen sind.

Bevorzugt sind Verbindungen der Formel (I), bei welchen

$R^1$ für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkoxycarbonylalkyl mit jeweils 1 bis 4 Kohlenstoffatomen in den einzelnen Alkylteilen, für geradkettiges oder verzweigtes Alkylthio mit 1 bis 6 Kohlenstoffatomen, für Cycloalkyl mit 3 bis 7 Kohlenstoffatomen, für Alkylamino mit 1 bis 6 Kohlenstoffatomen im geradkettigen oder verzweigten Alkylteil, für Alkoxyalkylamino mit jeweils 1 bis 4 Kohlenstoffatomen in den einzelnen geradkettigen oder verzweigten Alkylteilen oder für Dialkylamino mit jeweils 1 bis 6 Kohlenstoffatomen in den einzelnen geradkettigen oder verzweigten Alkylteilen, Cycloalkylamino mit 3 bis 6 Kohlenstoffatomen im Cycloalkylteil, oder für Amino steht, welches durch Cycloalkyl mit 3 bis 6 Kohlenstoffatomen, durch geradkettiges oder verzweigtes Alkyl mit 1 bis 4 Kohlenstoffatomen, durch geradkettiges oder verzweigtes Alkenyl mit 2 bis 6 Kohlenstoffatomen, durch geradkettiges oder verzweigtes Alkinyl mit 2 bis 6 Kohlenstoffatomen, durch geradkettiges oder verzweigtes Halogenalkyl mit 2 bis 6 Kohlenstoffatomen und bis zu 13 verschiedenen Halogenatomen, wobei Fluor, Chlor, Brom in Frage kommen, oder durch geradkettiges oder verzweigtes Alkoxycarbonylalkyl mit 1 bis 6 Kohlenstoffatomen in den einzelnen Alkylresten substituiert ist,

$R^2$ für Wasserstoff, für geradkettiges oder verzweigtes Alkyl mit 1 bis 6 Kohlenstoffatomen oder für Cycloalkyl mit 3 bis 7 Kohlenstoffatomen steht,

$R^3$ für Wasserstoff, Fluor, Chlor, Brom oder für geradkettiges oder verzweigtes Halogenalkyl mit 1 bis 4 Kohlenstoffatomen und 1 bis 9 gleichen oder verschiedenen Halogenatomen steht und

X für Sauerstoff oder Schwefel steht.

Besonders bevorzugt sind Verbindungen der Formel (I), bei welchen

$R^1$ für Methyl, Ethyl, n- oder i-Propyl, n-, i-, s- oder t-Butyl, für Chlormethyl, Chlorethyl, Bromethyl, Brommethyl, Dichlormethyl, Trichlormethyl oder Trifluormethyl, für Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, für Methylthio, Ethylthio, n- oder i-Propylthio, für Cyclopropyl, Cyclopentyl oder Cyclohexyl, für Methylamino, Ethylamino, n- oder i-Propylamino, n-, i-, s- oder t-Butylamino, Methoxymethylamino, Ethoxymethylamino, Methoxyethylamino, Ethoxyethylamino, oder für Dimethylamino, Diethylamino, Di-n-propylamino, Di-i-propylamino, Di-n-butylamino, Di-i-butylamino, Di-s-butylamino, N-Methyl-N-t-Butyl-amino, Cyclohexylamino oder N-Methyl-N-Cyclohexylamino steht,

$R^2$ für Wasserstoff, Methyl, Ethyl, n- oder i-Propyl, n-, i-, s- oder t-Butyl, für Cyclopropyl, Cyclopentyl oder Cyclohexyl steht,

$R^3$ für Wasserstoff, Fluor, Chlor oder für Halogenalkyl mit 1 bis 2 Kohlenstoffatomen und 1 bis 5 gleichen oder verschiedenen Halogenatomen steht und

X für Sauerstoff oder Schwefel steht.

Ganz besonders bevorzugt sind Verbindungen der Formel (I), bei welchen

$R^1$ für Methyl, Ethyl, n- oder i-Propyl, Chlormethyl, Dichlormethyl, Methoxymethyl, Methoxycarbonylmethyl, Methoxycarbonylethyl, Methylthio, Ethylthio, Cyclopropyl, Methylamino, Ethylamino, n- oder i-Propylamino, Methoxymethylamino, Dimethylamino, Diethylamino, Di-n-propylamino, Di-i-propylamino, N-Methyl-N-t-Butylamio, Cyclohexylamino oder N-Methyl-N-Cyclohexylamino steht,

$R^2$ für Wasserstoff, Methyl, Ethyl, n- oder i-Propyl oder Cyclopropyl steht,

$R^3$ für Wasserstoff, Chlor oder Trifluormethyl steht und

X für Sauerstoff steht.

Verwendet man beispielsweise 2-Methylamino-7-chlorbenzthiazol und Methylisocyanat als Ausgangsstoffe, so läßt sich der Reaktionsablauf des erfindungsgemäßen Verfahrens durch das folgende Formelschema darstellen:

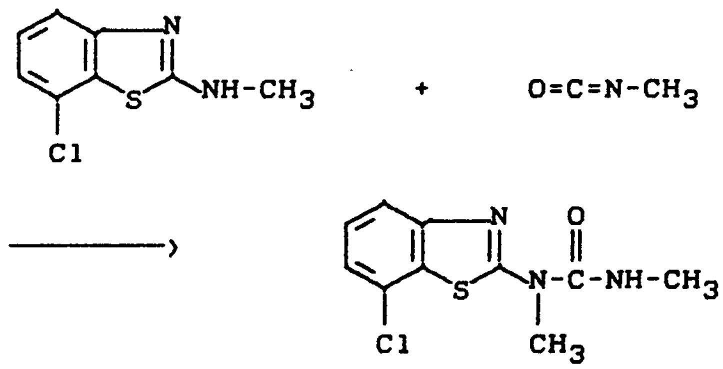

Die zur Durchführung des erfindungsgemäßen Verfahrens als Ausgangsstoffe benötigten 2-Amino-7-chlorbenzthiazole sind durch die Formel (II) allgemein definiert. In dieser Formel (II) stehen $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die 2-Amino-7-chlorbenzthiazole der Formel (II) sind teilweise bekannt (vgl. z.B. Arch. Pharm. 304, 687-695 [1971], DE 3522941; J. Med. Chem. 30, 465-473 [1987]; DE 3231885; DE 2801991; J. Heterocycl. Chem. 8, 309-310 [1971]).

Man erhält sie, wenn man 7-Chlor-2-alkyloxycarbonylbenzthiazolyl-N-oxide der Formel (V),

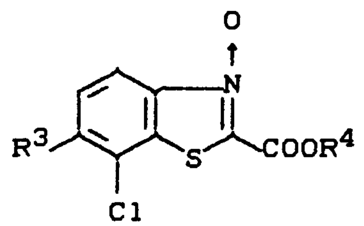

(V)

in welcher
$R^4$ für Alkyl steht und
$R^3$ die oben angegebene Bedeutung hat,
mit Natronlauge in allgemein üblicher Art und Weise verseift und anschließend decarboxyliert, dann die so erhältlichen Benzthiazolyl-N-oxide der Formel (VI),

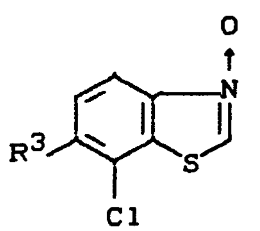

(VI)

in welcher
$R^3$ die oben angegebene Bedeutung hat,
mit einem Chlorierungsmittel wie beispielsweise Thionylchlorid oder Phosphorylchlorid oder Sulfurylchlorid umsetzt und schließlich die so erhältlichen 2,7-Dichlorbenzthiazole der Formel (VII),

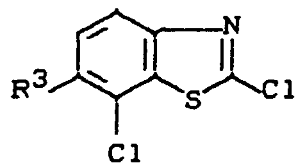

(VII)

in welcher
$R^3$ die oben angegebene Bedeutung hat,
mit primären Aminen der Formel (VIII),

$R^2$-$NH_2$ (VIII)

in welcher
$R^2$ die oben angegebene Bedeutung hat,
gegebenenfalls in Gegenwart eines Verdünnungsmittels wie beispielsweise Dimethylformamid und gegebenenfalls in Gegenwart eines Reaktionshilfsmittels wie beispielsweise überschüssiges Amin der Formel (VIII) bei Temperaturen zwischen 0° und 80° C umsetzt (vgl. auch die Herstellungsbeispiele).

7-Chlor-2-alkyloxycarbonylbenzthiazolyl-N-oxide der Formel (V) sind bekannt (vgl. z.B. EP 334138; EP 334134).

Primäre Amine der Formel (VIII) sind allgemein bekannte Verbindungen der organischen Chemie.

Die zur Durchführung des erfindungsgemäßen Verfahrens weiterhin als Ausgangsstoffe benötigten Acylhalogenide sind durch die Formel (III) allgemein definiert. In dieser Formel (III) stehen $R^1$ und X vorzugsweise für diejenigen Reste, die bereits im Zusammenhang mit der Beschreibung der erfindungsgemäßen Stoffe der Formel (I) als bevorzugt für diese Substituenten genannt wurden.

Hal steht vorzugsweise für Chlor oder Brom, insbesondere für Chlor.

Die Acylhalogenide der Formel (III) sind ebenfalls allgemein bekannte Verbindungen der organischen Chemie.

Die zur Durchführung des erfindungsgemäßen Verfahrens alternativ als Ausgangsstoffe benötigten Iso(thio)cyanate sind durch die Formel (IV) allgemein definiert.

In dieser Formel (IV) steht X vorzugsweise für diejenigen Reste, die bereits im Zusammenhang mit der Beschreibung der erfindungsgemäßen Stoffe der Formel (I) als bevorzugt für diese Substituenten genannt wurden.

$R^{1-1}$ steht vorzugsweise für geradkettiges oder verzweigtes Alkyl mit 1 bis 6, insbesondere mit 1 bis 4 Kohlenstoffatomen, ganz besonders für Methyl, Ethyl, n- oder i-Propyl.

Die Iso(thio)cyanate der Formel (IV) sind ebenfalls allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Das erfindungsgemäße Verfahren wird gegebenenfalls in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0° C und 150° C, vorzugsweise bei Temperaturen zwischen 20° C und 120° C.

Zur Durchführung des erfindungsgemäßen Verfahrens setzt man pro Mol an 2-Amino-7-chlorbenzthiazol der Formel (II) im allgemeinen 1 bis 2 Mol, vorzugsweise 1 bis 1.2 Mol an Acylhalogenid oder Iso(thio)-

cyanat der Formel (III) bzw. (IV) und gegebenenfalls 1 bis 2 Mol, vorzugsweise 1 bis 1.2 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen</u>: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

<u>Dikotyle Kulturen der Gattungen</u>: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen</u>: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen</u>: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Verbindungen mit besonders gutem Erfolg zur selektiven Bekämpfung von dikotylen Unkräutern insbesondere in monokotylen Kulturen wie beispielsweise Weizen einsetzen.

In entsprechenden Aufwandmengen besitzen die erfindungsgemäßen Wirkstoffe darüber hinaus auch insektizide, akarizide und fungizide Wirksamkeiten und lassen sich beispielsweise zur Bekämpfung von Phytophthora-, Venturia- und Pyricularia-Arten als Krankheitserreger in Gemüse-, Obst- und Reiskulturen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl,

Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-aminol-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxyl-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-aminol-carbonyl]-amino]-sulfonyl]-thiophen-2-carbonsäure-methylester (THIAMETURON) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

<u>Herstellungsbeispiele:</u>

Beispiel 1:

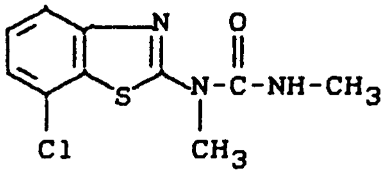

Zu 3.96 g (0.02 Mol) 2-Methylamino-7-chlorbenzthiazol in 50 ml Acetonitril gibt man 2 Tropfen Triethylamin und 1.25 g (0.022 Mol) Methylisocyanat und erhitzt 1 Stunde auf Rückflußtemperatur. Die erkaltete Reaktionsmischung wird abgesaugt und der Rückstand getrocknet.

Man erhält 3.6 g (71% der Theorie) an N,N'-Dimethyl-N-(7-chlor-2-benzthiazolyl)harnstoff vom Schmelzpunkt 176° C.

Herstellung der Ausgangsverbindungen

Beispiel II-1

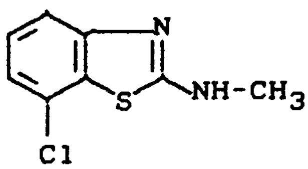

Zu 10.2 g (0.05 Mol) 2,7-Dichlorbenzthiazol in 50 ml Dimethylformamid gibt man bei 25° bis 30° C 15.5 g (0.15 Mol) 30%ige wäßrige Methylaminlösung und rührt eine Stunde bei dieser Temperatur. Zur Aufarbeitung gießt man die Reaktionsmischung in 300 ml Wasser, saugt ausgefallenen Feststoff ab, wäscht mit Wasser nach und trocknet über Phosphorpentoxid im Vakuum,

Man erhält 9.5 g (96% der Theorie) an 7-Chlor-2-methylaminobenzthiazol vom Schmelzpunkt 159-160° C.

Beispiel VII-1

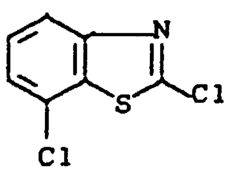

Zu 18.55 g (0.1 Mol) 7-Chlorbenzthiazol-N-oxid in 100 ml Chloroform gibt man tropfenweise unter Rühren 30.7 g (0.2 Mol) Phosphoroxychlorid, rührt anschließend bis zum Ende der Gasentwicklung bei Rückflußtemperatur. Zur Aufarbeitung wird nach ca. 2 Stunden im Vakuum eingeengt, der Rückstand in Dichlormethan aufgenommen und über Kieselgel chromatographiert (Laufmittel: Dichlormethan/Petrolether 1:1).

Man erhält 14 g (66% der Theorie) an 2,7-Dichlorbenzthiazol vom Schmelzpunkt 45-47° C.

Beispiel VI-1

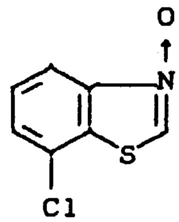

Zu 121.8 g (0.5 Mol) 7-Chlor-2-methoxycarbonylbenzthiazol-N-oxid (vgl. EP-A 334 138; EP-A 334 134) in einem Gemisch aus 2 l Methanol und 2 l Wasser tropft man bei 30° C bis 35° C unter Rühren 49 g (0.55 Mol) 45%ige Natronlauge, rührt anschließend eine Stunde bei Raumtemperatur, tropft dann innerhalb einer Stunde bei 30° C bis 40° C 61.3 g (0.6 Mol) Schwefelsäure in 200 ml Wasser gelöst zu und rührt 16 Stunden bei Raumtemperatur. Zur Aufarbeitung gibt man 1500 g Eis zu, saugt ausgefallenen Feststoff ab, wäscht mit Wasser nach und trocknet an der Luft.

Man erhält 80 g (86% der Theorie) an 7-Chlorbenzthiazol-N-oxid vom Schmelzpunkt >250° C.

In entsprechender Weise und gemäß den allgemeinen Angaben zur Herstellung erhält man die folgenden 2-Acylamino-7-chlorbenzthiazole der allgemeinen Formel (I):

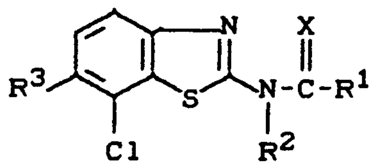

(I)

Tabelle 1

| Bsp.-Nr. | $R^1$ | $R^2$ | $R^3$ | X | Schmelzpunkt bzw. $^1$H-NMR |
|---|---|---|---|---|---|
| 2 | $-NH-CH_3$ | $C_2H_5$ | H | O | 148° C |
| 3 | $-NH-CH_3$ | $CH_3$ | H | S | 158° C |
| 4 | $-NH-CH_3$ | 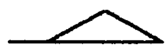 | H | O | 164° C |
| 5 | 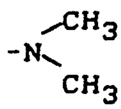 | $CH_3$ | Cl | O | 139° C |
| 6 | $-NH-CH_3$ | H | H | S | 227° C |
| 7 | $C_2H_5$ | H | H | O | 190° C |
| 8 | 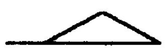 | $C_2H_5$ | H | O | 166° C |

Tabelle 1-Fortsetzung

| Bsp.-Nr. | $R^1$ | $R^2$ | $R^3$ | X | Schmelzpunkt bzw. $^1H$-NMR |
|---|---|---|---|---|---|
| 9 | $C_2H_5$ | $C_2H_5$ | H | O | 127° C |
| 10 | $-NH-CH(CH_3)_2$ | H | Cl | O | >250° C |
| 11 | $C_2H_5$ | $CH_3$ | Cl | O | 134° C |
| 12 | $-NH-CH_3$ | H | Cl | O | >250° C |
| 13 | 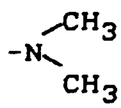 | H | H | O | 190° C |
| 14 | 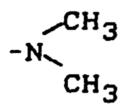 | $CH_3$ | H | O | 118° C |
| 15 | $-CH_2-CH(CH_3)_2$ | H | H | O | 167° C |
| 16 | $-CH(CH_3)_2$ | H | H | O | 186° C |
| 17 | $C_2H_5$ | $CH_3$ | H | O | 160° C |
| 18 | $-CH(CH_3)_2$ | H | Cl | O | 241° C |
| 19 | $CH_3$ | H | H | O | >250° C |
| 20 | $-NH-CH(CH_3)_2$ | $CH_3$ | H | O | 116° C |
| 21 | $-CH_2-CH(CH_3)_2$ | $CH_3$ | H | O | 110-111° C |
| 22 | $-NH-C_2H_5$ | $CH_3$ | H | O | 118° C |
| 23 | $-NH-CH_3$ | H | H | O | >250° C |
| 24 | $-NH-(CH_2)_2-CH_3$ | $CH_3$ | H | O | 85° C |
| 25 | $-NH-CH(CH_3)_2$ | $CH_3$ | Cl | O | 172° C |
| 26 | $-NH-(CH_2)_3-CH_3$ | 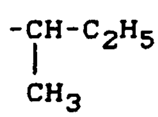 | H | O | Öl $^1H$-NMR: 9.3; 4.48; 3.35 |
| 27 | $-NH-(CH_2)_3-CH_3$ | $CH_3$ | H | O | 87° C |

Tabelle 1-Fortsetzung

| Bsp.-Nr. | $R^1$ | $R^2$ | $R^3$ | X | Schmelzpunkt bzw. $^1H$-NMR |
|---|---|---|---|---|---|
| 28 | 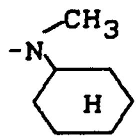 | $CH_3$ | H | O | 83-84° C |
| 29 | 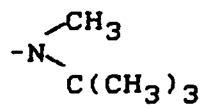 | $CH_3$ | H | O | 76-77° C |
| 30 | 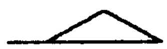 | H | Cl | O | >250° C |
| 31 | 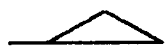 | $CH_3$ | H | O | 204° C |
| 32 | $-NH-(CH_2)_3-CH_3$ | H | Cl | O | 185° C |
| 33 | $C_2H_5$ | H | Cl | O | 241° C |
| 34 | $-NH-(CH_2)_2-CH_3$ | H | Cl | O | >250° C |
| 35 | $C_2H_5$ | $-CH(CH_3)_2$ | H | O | 87° C |
| 36 | $-C(CH_3)_3$ | H | H | O | 169° C |
| 37 | $-NH-CH_3$ | $-CH(CH_3)_2$ | H | O | 115° C |
| 38 | $-NH-C(CH_3)_3$ | $CH_3$ | H | O | 167° C |
| 39 | 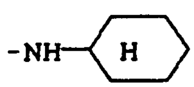 | $CH_3$ | H | O | 104° C |
| 40 | 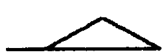 | H | H | O | 212° C |
| 41 | $-NH-CH_3$ | $CH_3$ | Cl | O | 221° C |
| 42 | $-S-C_2H_5$ | $CH_3$ | Cl | O | |
| 43 | $-S-C_2H_5$ | H | Cl | O | |

Tabelle 1-Fortsetzung

| Bsp.-Nr. | $R^1$ | $R^2$ | $R^3$ | X | Schmelzpunkt bzw. $^1H$-NMR |
|---|---|---|---|---|---|
| 44 | 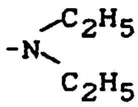 | $CH_3$ | Cl | O | |
| 45 | 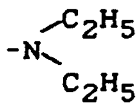 | H | H | O | |
| 46 | $-NH-CH_2-OCH_3$ | 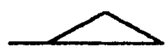 | H | O | 103° C |
| 47 | $-CH_2Cl$ | H | H | O | 184° C |
| 48 | $-CH_2Cl$ | $C_2H_5$ | H | O | 126° C |
| 49 | $-CH_2-OCH_3$ | H | Cl | O | 187° C |
| 50 | $-NH-CH_2-OCH_3$ | $CH_3$ | Cl | O | 162° C |
| 51 | $-NH-CH_2-OCH_3$ | $CH_3$ | H | O | 116° C |
| 52 | $-CH_2CH_2-COOCH_3$ | H | H | O | >86° C |
| 53 | $-CH_2CH_2-COOCH_3$ | H | Cl | O | 185° C |
| 54 | $-CH_2-COOCH_3$ | H | Cl | O | 204° C |
| 55 | $-CH_2-OCH_3$ | H | H | O | 129° C |

Tabelle 1-Fortsetzung

| Bsp.-Nr. | $R^1$ | $R^2$ | $R^3$ | X | Schmelzpunkt bzw. $^1H$-NMR |
|---|---|---|---|---|---|
| 56 | 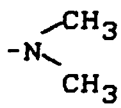 | $-C_2H_5$ | H | O | Öl |
| 57 | 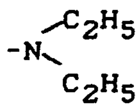 | $-C_2H_5$ | H | O | Öl |
| 58 | $-CH_2-Cl$ | $-CH(CH_3)_2$ | H | O | Fp. 94° C |
| 59 | $-NH-CH_2-CH_2-OCH_3$ | $-CH_3$ | H | O | Fp. 99° C |
| 60 | $-NH-(CH_2)_3-OC_2H_5$ | $-CH_3$ | H | O | Fp.98-99° C |
| 61 | $-NH-CH_2-CF_3$ | $-CH_3$ | H | O | Fp. 120° C |
| 62 | $-NH-(CH_2)_3-OC_2H_5$ | H | Cl | O | Fp. 156° C |
| 63 | $-S-C_2H_5-$ | $-CH_3$ | H | O | Fp. 131° C |
| 64 | 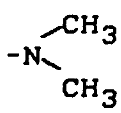 | $-CH_3$ | H | S | Fp. 92° C |
| 65 | 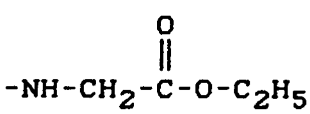 | $-CH_3$ | H | O | Fp. 118° C |
| 66 | 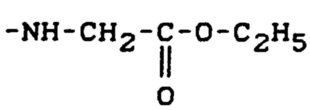 | H | Cl | O | Fp.>250° C |

Tabelle 1-Fortsetzung

| Bsp.-Nr. | $R^1$ | $R^2$ | $R^3$ | X | Schmelzpunkt bzw. $^1H$-NMR |
|---|---|---|---|---|---|
| 67 | $-NH-(CH_2)_3-O-C_2H_5$ | 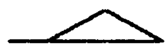 | H | O | Öl |
| 68 | 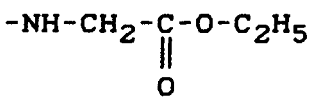 | $-CH_3$ | Cl | O | Fp. 172° C |
| 69 | 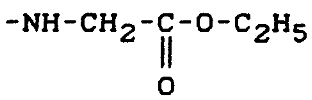 | H | H | O | Fp. 178° C |
| 70 | $-NH_2$ | $-CH_3$ | H | O | Fp. 200° C |
| 71 | 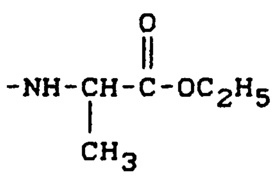 | $-CH_3$ | H | O | Fp. 87° C |
| 72 | 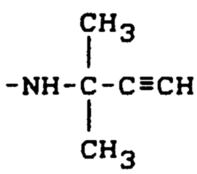 | H | H | O | Fp. 134° C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiel:

In dem folgenden Anwendungsbeispiel wurde die nachstehend aufgeführte Verbindung als Vergleichssubstanz eingesetzt:

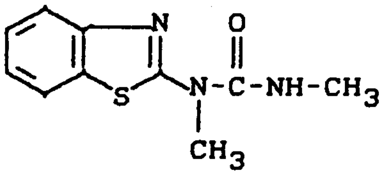

(A)

N,N'-Dimethylbenzthiazol-2-ylharnstoff (bekannt aus GB 1085430 [1966]).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber der Vergleichssubstanz (A) zeigen in diesem Test z.B. die Verbindungen gemäß folgender Herstellungsbeispiele: 1 und 2.

**Patentansprüche**

**1.** 2-Acylamino-7-chlorbenzthiazole der Formel (I)

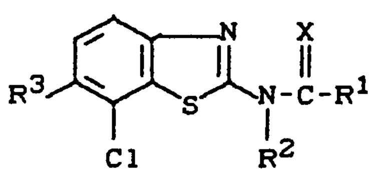

(I)

in welcher

$R^1$ für Alkyl, Halogenalkyl, Alkoxyalkyl, Alkoxycarbonylalkyl, Cycloalkyl, Alkylthio, Dialkylamino oder für Amino steht, welches durch Cycloalkyl, Alkyl, Alkoxyalkyl, Halogenalkyl, Alkenyl, Alkinyl und/oder Alkoxycarbonylalkyl substituiert ist,

$R^2$ für Wasserstoff, Alkyl oder Cycloalkyl steht,

$R^3$ für Wasserstoff, Halogen oder Halogenalkyl steht und

X für Sauerstoff oder Schwefel steht.

**2.** 2-Acylamino-7-chlorbenzthiazole der Formel (I) gemäß Anspruch 1, in welcher

$R^1$ für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkoxycarbonylalkyl mit jeweils 1 bis 4 Kohlenstoffatomen in den einzelnen Alkylteilen, für

geradkettiges oder verzweigtes Alkylthio mit 1 bis 6 Kohlenstoffatomen, für Cycloalkyl mit 3 bis 7 Kohlenstoffatomen, für Alkylamino mit 1 bis 6 Kohlenstoffatomen im geradkettigen oder verzweigten Alkylteil, für Alkoxyalkylamino mit jeweils 1 bis 4 Kohlenstoffatomen in den einzelnen geradkettigen oder verzweigten Alkylteilen oder für Dialkylamino mit jeweils 1 bis 6 Kohlenstoffatomen in den einzelnen geradkettigen oder verzweigten Alkylteilen, Cycloalkylamino mit 3 bis 6 Kohlenstoffatomen im Cycloalkylteil, oder für Amino steht, welches durch Cycloalkyl mit 3 bis 6 Kohlenstoffatomen, durch geradkettiges oder verzweigtes Alkyl mit 1 bis 4 Kohlenstoffatomen, durch geradkettiges oder verzweigtes Alkenyl mit 2 bis 6 Kohlenstoffatomen, durch geradkettiges oder verzweigtes Alkinyl mit 2 bis 6 Kohlenstoffatomen, durch geradkettiges oder verzweigtes Halogenalkyl mit 2 bis 6 Kohlenstoffatomen und bis zu 13 verschiedenen Halogenatomen, wobei Fluor, Chlor, Brom in Frage kommen, oder durch geradkettiges oder verzweigtes Alkoxycarbonylalkyl mit 1 bis 6 Kohlenstoffatomen in den einzelnen Alkylresten substituiert ist,

$R^2$ für Wasserstoff, für geradkettiges oder verzweigtes Alkyl mit 1 bis 6 Kohlenstoffatomen oder für Cycloalkyl mit 3 bis 7 Kohlenstoffatomen steht,

$R^3$ für Wasserstoff, Fluor, Chlor, Brom oder für geradkettiges oder verzweigtes Halogenalkyl mit 1 bis 4 Kohlenstoffatomen und 1 bis 9 gleichen oder verschiedenen Halogenatomen steht und

X für Sauerstoff oder Schwefel steht.

**3.** 2-Acylamino-7-chlorbenzthiazole der Formel (I) gemäß Anspruch 1, in welcher

$R^1$ für Methyl, Ethyl, n- oder i-Propyl, n-, i-, s-oder t-Butyl, für Chlormethyl, Chlorethyl, Bromethyl, Brommethyl, Dichlormethyl, Trichlormethyl oder Trifluormethyl, für Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, für Methylthio, Ethylthio, n-oder i-Propylthio, für Cyclopropyl, Cyclopentyl oder Cyclohexyl, für Methylamino, Ethylamino, n-oder i-Propylamino, n-, i-, s- oder t-Butylamino, Methoxymethylamino, Ethoxymethylamino, Methoxyethylamino, Ethoxyethylamino, oder für Dimethylamino, Diethylamino, Di-n-propylamino, Di-i-propylamino, Di-n-butylamino, Di-i-butylamino, Di-s-butylamino, N-Methyl-N-t-Butyl-amino, Cyclohexylamino oder N-Methyl-N-Cyclohexylamino steht,

$R^2$ für Wasserstoff, Methyl, Ethyl, n- oder i-Propyl, n-, i-, s- oder t-Butyl, für Cyclopropyl, Cyclopentyl oder Cyclohexyl steht,

$R^3$ für Wasserstoff, Fluor, Chlor oder für Halogenalkyl mit 1 bis 2 Kohlenstoffatomen und 1 bis 5 gleichen oder verschiedenen Halogenatomen steht und

X für Sauerstoff oder Schwefel steht.

**4.** 2-Acylamino-7-chlorbenzthiazole der Formel (I) gemäß Anspruch 1, in welcher

$R^1$ für Methyl, Ethyl, n- oder i-Propyl, Chlormethyl, Dichlormethyl, Methoxymethyl, Methoxycarbonylmethyl, Methoxycarbonylethyl, Methylthio, Ethylthio, Cyclopropyl, Methylamino, Ethylamino, n- oder i-Propylamino, Methoxymethylamino, Dimethylamino, Diethylamino, Di-n-propylamino, Di-i-propylamino, N-Methyl-N-t-Butylamio, Cyclohexylamino oder N-Methyl-N-Cyclohexylamino steht.

$R^2$ für Wasserstoff, Methyl, Ethyl, n- oder i-Propyl oder Cyclopropyl steht,

$R^3$ für Wasserstoff, Chlor oder Trifluormethyl steht und

X für Sauerstoff steht.

**5.** Verfahren zur Herstellung von 2-Acylamino-7-chlorbenzthiazolen der Formel (I)

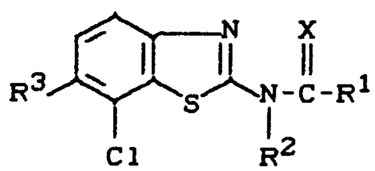

(I)

in welcher

$R^1$ für Alkyl, Halogenalkyl, Alkoxyalkyl, Alkoxycarbonylalkyl, Cycloalkyl, Alkylthio, Alkylamino, Alkoxyalkylamino, Dialkylamino, für Cycloalkylamino oder für Amino steht, welches durch

Cycloalkyl und durch Alkyl substituiert ist,

$R^2$ für Wasserstoff, Alkyl oder Cycloalkyl steht,

$R^3$ für Wasserstoff, Halogen oder Halogenalkyl steht und

X für Sauerstoff oder Schwefel steht,

dadurch gekennzeichnet, daß man 2-Amino-7-chlorbenzthiazole der Formel (II),

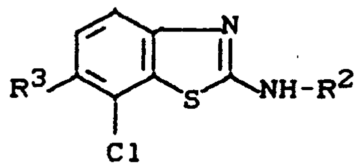

(II)

in welcher

$R^2$ und $R^3$ die oben angegebene Bedeutung haben,

mit Acylhalogeniden der Formel (III),

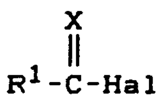

(III)

in welcher

Hal für Halogen steht und

$R^1$ und X die oben angegebene Bedeutung haben,

oder mit Iso(thio)cyanaten der Formel (IV),

$R^{1-1}$-N=C=X (IV)

in welcher

$R^{1-1}$ für Alkyl steht und

X die oben angegebene Bedeutung hat,

gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einem 2-Acylamino-7-chlorbenzthiazol der Formel (I) gemäß Anspruch 1 oder 5.

**7.** Verfahren zur Bekämpfung von unerwünschten Pflanzen, dadurch gekennzeichnet, daß man 2-Acylamino-7-chlorbenzthiazole der Formel (I) gemäß Anspruch 1 oder 5 auf die unerwünschten Pflanzen und/oder ihren Lebensraum einwirken läßt.

**8.** Verwendung von 2-Acylamino-7-chlorbenzthiazolen der Formel (I) gemäß Anspruch 1 oder 5 zur Bekämpfung von unerwünschten Pflanzen.

**9.** Verfahren zur Herstellung von herbiziden Mitteln, dadurch gekennzeichnet, daß man 2-Acylamino-7-chlorbenzthiazole gemäß Anspruch 1 oder 5 mit Streckmitteln und/oder oberflächenaktiven Substanzen vermischt.

Europäisches Patentamt

# EUROPÄISCHER RECHERCHENBERICHT

Nummer der Anmeldung

**EP 91 11 0326**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 431 801 (NIPPON SODA CO.,LTD.)<br>* Seite 1 - Absatz 2 * | 1,6 | C 07 D 277/82<br>A 01 N 43/78 |
| A | DE-A-2 020 300 (FARBENFABRIKEN BAYER AG.)<br>* Seiten 1 - 2, Absatz 1 * | 1,6 | |
| A | DE-A-2 014 565 (BADISCHE ANILIN- UND SODA-FABRIK AG.)<br>* Seite 1 * | 1,6 | |
| A | DE-A-1 953 357 (FARBENFABRIKEN BAYER AG.)<br>* Seiten 1 - 2, Zeile 1 * | 1,6 | |
| A | US-A-4 319 914 (LEONARD J.STACH)<br>* Spalte 1 - Zeile 33 * | 1,6 | |

RECHERCHIERTE SACHGEBIETE (Int. Cl.5)

C 07 D 277/00

Der vorliegende Recherchenbericht wurde für alle Patentansprüche erstellt

| Recherchenort | Abschlußdatum der Recherche | Prüfer |
|---|---|---|
| Berlin | 16 Oktober 91 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument